# EUROPEAN PATENT SPECIFICATION

(11) **EP 4 101 438 B1**
(45) Date of publication and mention of the grant of the patent: **16.04.2025**
(21) Application number: 21178961.5
(22) Date of filing: 11.06.2021
(51) Int. Cl.: A61K 31/4184, A61K 9/107, A61K 9/127, A61K 31/155, A61K 9/51, A61K 31/7032, A61K 31/704, A61K 45/06, A61P 35/00, A61K 9/00, A61K 9/14, A61K 9/20

(54) **COMBINATION TREATMENT OF A SAPONIN AGENT AND AN ANTHELMINITIC AGENT AGAINST CANCER**
KOMBINATIONSBEHANDLUNG EINES SAPONINS UND EINES ANTHELMINITIKUMS GEGEN KREBS
TRAITEMENT COMBINÉ D'UN AGENT SAPONINE ET D'UN AGENT ANTHELMINITIQUE CONTRE LE CANCER

(43) Date of publication of application: 14.12.2022
(73) Proprietor: RainCastle bio. Limited, London WC2H 9JQ (GB)
(72) Inventor: ALENEZE, Yousef M A KH M, Salam (KW); AL-AKKAD, Walid, London WC2H 9JQ (GB)
(74) Representative: Thoma, Michael

(56) References cited:
- WO-A1-2004/056379
- WO-A1-2015/021922
- WO-A1-2019/109074
- CN-A- 111 759 853
- US-A1- 2014 199 296
- US-A1- 2019 125 690
- TROP J PHARM ET AL: "Popovic et al Application of a widely-used tropical anti-worm agent, mebendazole, in modern oncology", TROPICAL JOURNAL OF PHARMACEUTICAL RESEARCH OCTOBER, 1 October 2017 (2017-10-01), pages 2555, XP055525741, Retrieved from the Internet <URL:https://www.ajol.info/index.php/tjpr/article/view/162964> [retrieved on 20211117], DOI: 10.4314/tjpr.v16i10.32
- HONG HEEOK ET AL: "Anticancer Activities of Ginsenosides, the Main Active Components of Ginseng", EVIDENCE-BASED COMPLEMENTARY AND ALTERNATIVE MEDICINE, vol. 2021, 3 February 2021 (2021-02-03), US, pages 1 - 10, XP055861954, ISSN: 1741-427X, Retrieved from the Internet <URL:http://downloads.hindawi.com/journals/ecam/2021/8858006.xml> DOI: 10.1155/2021/8858006
- ABONGWA MELANIE ET AL: "A brief review on the mode of action of antinematodal drugs", ACTA VETERINARIA, vol. 67, no. 2, 1 June 2017 (2017-06-01), pages 137 - 152, XP093066828, DOI: 10.1515/acve-2017-0013
- JUANG YU-PU ET AL: "Biological and Pharmacological Effects of Synthetic Saponins", MOLECULES, vol. 25, no. 21, 1 November 2020 (2020-11-01), DE, pages 4974, XP055908604, ISSN: 1433-1373, DOI: 10.3390/molecules25214974
- STRBAC FILIP ET AL: "In vitro and in vivo anthelmintic efficacy of peppermint (Mentha x piperita L.) essential oil against gastrointestinal nematodes of sheep", FRONTIERS IN VETERINARY SCIENCE, vol. 10, 10 August 2023 (2023-08-10), Lausanne, XP093127271, ISSN: 2297-1769, DOI: 10.3389/fvets.2023.1232570
- PLOTKIN S. ET AL: "Hookworm Vaccines", CLINICAL INFECTIOUS DISEASES, vol. 46, no. 2, 15 January 2008 (2008-01-15), US, pages 282 - 288, XP093127277, ISSN: 1058-4838, Retrieved from the Internet <URL:https://watermark.silverchair.com/46-2-282.pdf?token=AQECAHi208BE49Ooan9kkhW_Ercy7Dm3ZL_9Cf3qfKAc485ysgAAA14wggNaBgkqhkiG9w0BBwagggNLMIIDRwIBADCCA0AGCSqGSIb3DQEHATAeBglghkgBZQMEAS4wEQQMSoVgUk0aX_GFFTl6AgEQgIIDEW3ohU2bevvQxGn8AzIEreR0yCa3MV6SNnS0REAjGOU0xFyV0QlGx-JXIBlrlS96Fyw5HWP3J83t7Kg6c97dLGKw3bq> DOI: 10.1086/524070
- LEE SUI-TING ET AL: "Alpha-tomatine synergises with paclitaxel to enhance apoptosis of androgen-independent human prostate cancer PC-3 cells in vitro and in vivo", PHYTOMEDICINE, vol. 20, no. 14, 1 November 2013 (2013-11-01), AMSTERDAM, NL, pages 1297 - 1305, XP093128514, ISSN: 0944-7113, DOI: 10.1016/j.phymed.2013.07.002
- ELEKOFEHINTI OLUSOLA OLALEKAN ET AL: "Saponins in Cancer Treatment: Current Progress and Future Prospects", PATHOPHYSIOLOGY, vol. 28, no. 2, 5 June 2021 (2021-06-05), pages 250 - 272, XP093132057, ISSN: 1873-149X, DOI: 10.3390/pathophysiology28020017
- ZHOU PIAO ET AL: "Saikosaponin D: review on the antitumour effects, toxicity and pharmacokinetics", PHARMACEUTICAL BIOLOGY, vol. 59, no. 1, 1 January 2021 (2021-01-01), NL, pages 1478 - 1487, XP093132061, ISSN: 1388-0209, DOI: 10.1080/13880209.2021.1992448
- ZHU S ET AL: "Anthelmintic activity of saikosaponins a and d from radix bupleuri against Dactylogyrus spp. infecting goldfish", DISEASES OF AQUATIC ORGANISMS, vol. 111, no. 2, 30 September 2014 (2014-09-30), DE, pages 177 - 182, XP093132060, ISSN: 0177-5103, DOI: 10.3354/dao02789

## Description

The present invention relates to the field of treating cancer. In particular, the present disclosure relates to a combination treatment for cancer as well as compositions to be used in the treatment of cancer.

Many attempts to treat malignant neoplastic cells through conventional therapies have been met with limited success due to inter- and intra-tumor heterogeneity within the mosaic of tumor subclones. For example, US2014199296A1 discloses a cancer drug. Further prior art is the publication by Elekofehinti Olusola Olalekan ET AL: "Saponins in Cancer Treatment: Current Progress and Future Prospects", Pathophysiology, vol. 28, no. 2, 5 June 2021 (2021-06-05), pages 250-272, XP093132057, ISSN: 1873-149X, DOI: 10.3390/pathophysiology28020017

Multi-clonal tumors are major setbacks to moleclarly-targeted therapies (which only inhibit subsets of certain tumor clones that host the druggable oncogenic targets), thus rendering other competing tumor subclones (with undruggable oncogenic targets) resistant to such treatments.

Moreover, some tumor cells exhibit stem like properties with limited proliferation particularly upon exposure to treatment yet retain self-renewal capacity (cancer stem cells or CSCs) which could result in tumor relapse. CSCs could also migrate to distant organs and form distant metastatic tumors with more diverse tumor subclones, particularly under increased selective pressures of survival, thus certain emerging subclones will develop increased resistance to chemotherapy and molecular targeted therapy which will further progress to aggressive tumor progression, worse prognosis and poor survival.

From a patient point of view, chemotherapy leads to poor quality of life due to well-documented side effects, including cancer cachexia (muscle wasting and loss of appetite), associated with high pro-inflammatory TNF-alpha levels, chemotherapy induced alopecia due to non-specific targeting of high proliferative cells, fatigue and physical (including bone cancer pain) and neurological pain through IL-6-TRPA1 and TNF-alpha-TRPA1 pathways (Liu et al., 2019). Blocking IL-6 and TNF-alpha was shown to be beneficial in inhibiting pain in in-vivo models including chemotherapy-induced pain.

Thus, of great interest, was the paradigm shift towards antigen-independent immuno-oncology for cancer treatment to concurrently target different subclones of the heterogenous tumor mosaic simultaneously.

However, despite efforts of non-antigen specific immune-oncology (particularly immune checkpoint inhibitors ICIs) showing promise in achieving partial and even some complete responses in can-cer patients, a high risk of autoimmune-like immune related adverse events (iRAEs) commonly arises in patients. Persistence in T-cell responses, through in-hibiting T-cell exhaustion (via ICIs) could prolong the expression of inflammatory cytokines and could trigger self-antigen presentation alongside tumor antigen presentation; thus, targeting both tumor and surrounding inflamed target organ or tissue (König & Läubli, 2020). Post-mortem studies showed that melanoma cancer patients receiving ICIs led to immune infiltration in the myocardial tissue which developed into myocarditis (Gürdo 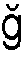 an, 2020).

Among the most common inflammatory cytokines as biomarkers of iRAEs is IL-6 which is also known to be a surrogate of immune response, inflammation, tumor progression and pain. Serum IL-6 is associated with worse prognosis and poor survival in cancer patients.

Among the first treatments of iRAEs such severe arthri-tis, myocarditis, uveitis, great vasculitis, severe pneumonia, great vasculitis, and myasthenia gravis is Tocilizumab (Anti IL-6 Receptor antibody) which inhibits IL-6 signalling. Targeting IL-6 pathway does not activate tumor progression.

Therefore, tumor cell killing without triggering IL-6 and/or activate IL-6 signalling pathways and other inflammatory cytokines pose as great benefit for patients such as inhibit-ing cancer cachexia, physical and neuropathic pain and fatigue- thus contributing towards improving quality of life.

The present invention is set out in the appended set of claims.

This invention presents the combination of Ginsenoside and Mebendazole to trigger non-inflammatory, antigen independent immune-mediated tumor cell killing without the secretion of IL-6 and TNF-alpha. Therefore, Ginsenoside and Mebendazole in combination reduce cytokine release commonly associated with cancer cachexia, physical and neuropathic pain, fatigue and poor quality of life.

Ginsenoside, a bioactive saponin compound found in Panax ginseng in trace amounts, poses anti-proliferative and pro-apoptotic effects in tumors. Ginsenoside contributes to both extrinsic apoptosis (via Death receptors such as FAS and TRAIL) and intrinsic mitochondrial apoptosis, resulting in caspase 8 and caspase 3/7 activation downstream.

Additionally, activated caspase 8 could also indirectly activate caspase 3/7 by truncating BID, a pro-apoptotic protein, into its functional form tBID; tBID then contributes to translocating BAX to the mitochondria to re-lease mitochondrial cytochrome c thus triggering intrinsic apoptosis as well.

With respect to extrinsic apoptosis, tumors generally live in harsh pathophysiological conditions such as low glucose and hypoxia which trigger intracellular ER stress and impairment in protein folding, lipid metabolism and calcium level regulation. Ginsenoside increases ER stress towards which expressing death receptors such as DR4 and DR5 receptors in tumors via PERK-ATF4-CHOP pathway; this primes cancer cells for extrinsic apoptosis upon interaction with cells that strongly express TRAIL (ligand) particularly, activated T cells and monocytes (Lam et al., 2020; Martín-Pérez et al., 2011).

However, there are several TRAIL-resistant cancer cells which are due to increased pro-survival anti-apoptotic proteins. Nevertheless, these resistant cells are sensitized to apoptosis upon inhibition of the pro-survival Akt- pathway (commonly expressed in many tumors) inhibition resulting in a de-crease of pro-survival proteins (such as Bcl-2 and BcL-xL) compared to pro-apototic counterparts (BAX).

Akt also phosphorylates BAX at Serine 184 residue (S184) which causes BAX to inhibit apoptosis. Ginsenosides inhibit Akt-pathway and disrupt plasma membrane lipid rafts (sphingolipid and cholesterol enriched micro domains of transmembrane proteins found clustered together which serve as external components of cell signalling pathways). This inactivates the pro-surivival Akt pathway and helps to rapidly and strongly translocate BAX into the mitochondria, thus further triggering mitochondrial-based intrinsic apoptosis.

To further sensitise TRAIL apoptosis, three strategies could be approached: (i) By further decreasing pro-survival proteins in tumors through further inhibition of pro-survival proteins. (ii) By increasing TRAIL-ligand expression in cells surrounding tumors, by enhancing immune activation. (iii) Convert non-immunological 'cold' tumors to immune-infiltrative hot tumors.

Mebendazole, an anti-helminth drug, addresses the three strategies above by (i) phosphorylating and deactivating BCL-2 protein, (ii) enhance T-cell activation through increased clustering and interaction between CD14+monocytes/macrophages and T-cells and (iii) re-polarize immunosuppressive tumor-promoting M2 macrophages to M1 classically activated antitumor macrophages which can directly kill the tumor and provide chemokine signals to increase immune infiltration of activated immune cells expressing TRAIL could interact with TRAIL-sensitized tumor cells to induce cancer cell death.

Hence in summary, antigen-independent tumor cell death is based on extrinsic death receptor-based and intrinsic mitochondrial-based apoptotic pathways, suggestively through direct interaction of death ligands in activated immune cells and death receptors in tumor cells; where ginsenosides up-regulate death receptors in stressed malnourished or hypoxic cells (such as tumor cells), while mebendazole enhances T-cell activation and limits intracellular anti-apoptotic proteins in cancer cells.

Rather than systemically targeting highly proliferative cells which results in off-target toxicities, such as chemotherapy-induced alopecia, selective tumor cell killing based upon cross-sectional ER stress-based apoptotic pathways among different tumors.

This provides a strategy to selectively target different malignant cells of different tissue origins while keeping physiologically unstressed cells intact and non-targeted, particularly hair follicles of cancer patients.

Our studies also incorporate immune mediated tumor cell killing in cancer cell lines that harbor other very common oncogenic mutations such as:
A549 Lung cancer cell line (CDKN2A mutant, K-RAS mutant)
H1299 Lung cancer cell line (TP53-null mutant, NRAS mutant)
MDA-MB-231 Breast cancer cell line (BRAF mutant, CDKN2A mutant, K-RAS mutant, TP53 mutant)
HCT-116 Colon cancer cell line (K-RAS mutant, PIK3CA mutant)
PANC-1 Pancreatic cancer cell line (K-RAS mutant, TP53 mutant, CDKN2A mutant)
SNU-449 Liver cancer cell line (TP53 mutant)

The present invention relates to a composition comprising a saponin agent, namely ginsenoside, and an anthelmintic agent, namely mebendazole, for use in the treatment of cancer, in particular in the treatment of solid tumors.

In the following, aspects of the present disclosure are described that may be realised individually or in combination in an embodiment or embodiments of the present invention.

A first aspect of the invention relates to a composition for use in a method of treating cancer, the composition comprising an effective amount of at least one saponin agent and an effective amount of at least one anthelmintic agent, wherein the saponin agent is a ginsenoside and the anthelmintic agent is a methyl N-(6-benzoyl-1H-benzimidazol-2-yl)carbamate (mebendazole).

A second aspect relates to the composition of aspect 1, further comprising an effective amount of at least one biguanide agent, wherein the biguanide agent preferably is N,N-dimethylbiguanide (metformin).

A third aspect relates to the composition of one of aspects 1-2, wherein the composition is a nanocarrier formulation, wherein the nanocarrier preferably is PEGylat-ed or non-PEGylated.

A fourth aspect relates to the composition of one of aspect 3, wherein the nanocarrier comprises SMEEDs, SNEDDS, SEDDS, solid lipid nanopartice, nanostruc-tured lipid carrier, microemulsions, liposome, micelles, polymeric nanoparticle, pol-ymeric micelle, dendrimer and / or mesoporous nanoparticles, amorphous solid dispersions, solid dispersions, micronised particles, hydrogels, dendrimers, cy-clodextrins, polymer drug conjugates, iron oxide nanoparticle (magnetic carrier), gold nanoparticle

A fifth aspect relates to the composition of one of aspects 1-4, wherein the cancer is selected from the group consisting of solid tumor and non-solid tumors and wherein preferably the cancer is a solid tumor selected from the group consist-ing of lung cancer, liver cancer, pancreatic cancer, colorectal cancer, breast can-cer, prostate cancer, brain cancer, stomach cancer, kidney cancer and cervical cancer.

An sixth aspect relates to the composition of one of aspects 1-5, wherein the saponin agent, namely a ginsenoside, is administered at an amount of from 0.001 mg/Kg body weight to 200 mg/Kg body weight, preferably from 0.01 mg/Kg body weight to 100 mg/Kg body weight, in particular from 0.1 mg/Kg body weight to 50 mg/Kg body weight (for mice and rats); and administered at an amount of from 0.001 mg/Kg body weight to 20 mg/kg body weight, preferably from 0.01 mg/Kg body weight to 15 mg/kg body weight, in particular from 0.01 mg/Kg body weight to 10 mg/kg body weight (for humans).

The anthelmintic agent, namely mebendazole, may also be administered at an amount of from 0.001 mg/Kg body weight to 200 mg/Kg body weight (for mice and rats); and administered at an amount of from 0.001 mg/Kg body weight to 20 mg/kg body weight (for humans) with the preferred dosage as recited above.

Similarly, the biguanide agent, preferably metformin, may also be administered at an amount of from 0.001 mg/Kg body weight to 200 mg/Kg body weight for (mice and rats); and administered at an amount of from 0.001 mg/Kg body weight to 20 mg/kg body weight (for humans) with the preferred dosage as recited above.

The saponin agent, the anthelmintic agent and / or the biguanide agent may be present at the same or at different amounts in a composition according to the present disclosure. The saponin agent, the anthelmintic agent and /or the biguanide agent may be administered at the same or at different amounts or dosages in a method of treatment according to the present disclosure.

Saponin maybe administered at an amount of from 0.001 mg/Kg body weight to 200 mg/Kg body weight (for mice and rats); and administered at an amount of from 0.001 mg/Kg body weight to 20 mg/kg body weight (for humans) in combination with antihelmentic agent and/or biguanide agent, each preferably administered at the same range of an amount of from 0.001 mg/Kg body weight to 200 mg/Kg body weight for mice and rats); and administered at an amount of from 0.001 mg/Kg body weight to 20 mg/kg body weight (for humans). Each component is preferably administered at an amount of from 0.001 mg/Kg body weight to 200 mg/Kg body weight, preferably from 0.01 mg/Kg body weight to 100 mg/Kg body weight, in particular from 0.1 mg/Kg body weight to 50 mg/Kg body weight (for mice and rats); and administered at an amount of from 0.001 mg/Kg body weight to 20 mg/kg body weight, preferably from 0.01 mg/Kg body weight to 15 mg/kg body weight, in particular from 0.01 mg/Kg body weight to 10 mg/kg body weight (for humans).

A seventh aspect relates to the composition of one of aspects 1-6, wherein the effective amount of at least one saponin agent, namely ginsenoside, and the effective amount of at least one anthelmintic agent, namely mebendazole, and / or the effective amount of at least one biguanide agent are present in a single formulation or are present in at least two separate formulations, wherein preferably the saponin agent, i.e. the ginsenoside, is administered at an amount of from 0.001 mg/Kg body weight to 200 mg/Kg body weight (for mice and rats); and administered at an amount of from 0.001 mg/Kg body weight to 20 mg/kg body weight (for humans). The saponin agent is preferably administered at an amount of from 0.001 mg/Kg body weight to 200 mg/Kg body weight, preferably from 0.01 mg/Kg body weight to 100 mg/Kg body weight, in particular from 0.1 mg/Kg body weight to 50 mg/Kg body weight (for mice and rats); and administered at an amount of from 0.001 mg/Kg body weight to 20 mg/kg body weight, preferably from 0.01 mg/Kg body weight to 15 mg/kg body weight, in particular from 0.01 mg/Kg body weight to 10 mg/kg body weight (for humans).

The anthelmintic agent, i.e. mebendazole, may also be administered at an amount of from 0.001 mg/Kg body weight to 200 mg/Kg body weight for mice and rats); and administered at an amount of from t0.001 mg/Kg body weight to 20 mg/kg body weight (for humans).

Similarly, the biguanide agent, preferably metformin, may also be administered at an amount of from 0.001 mg/Kg body weight to 200 mg/Kg body weight for (mice and rats); and administered at an amount of from 0.001 mg/Kg body weight to 20 mg/kg body weight (for humans)

A eighth aspect relates to the composition of one of aspects 1-7, wherein the effective amount of at least one saponin agent, i.e. ginsenoside, and the effective amount of at least one anthelmintic agent, i.e. mebendazole, and / or the effective amount of at least one biguanide agent are administered sequentially or concurrently.

The present disclosure also encompasses the aspect of a method of preparation of a composition according to one of the preceding aspects.

Another, nineth, aspect of the disclosure relates to a method of treating cancer, the method comprising administering to a subject in need thereof an effective amount of at least one saponin agent, namely ginsenoside, and an effective amount of at least one anthelmintic agent, namely mebendazole.

The saponin agent and the anthelmintic agent can be administered simultaneously or sequentially and / or can be administered in a single composition or multiple separate compositions.

The subject is preferably human. The cancer can be a cancer comprising cancer stem cells.

A tenth aspect relates to a method according to aspect 9, wherein the method further comprises administering to the subject an effective amount of at least one biguanide agent, wherein the biguanide agent is N,N-dimethylbiguanide (metformin).

An eleventh aspect relates to a method according to one of aspects 9 to 10, wherein the composition is a nanocarrier formulation, wherein the nanocarrier preferably is PEGylated or non-PEGylated.

A twelth aspect relates to a method according to one of aspect 11, wherein the nanocarrier comprises SMEEDs, SNEDDS, SEDDS, solid lipid nanopartice, nanostructured lipid carrier, microemulsions, liposome, micelles, polymeric nanoparticle, polymeric micelle, dendrimer and / or mesoporous nanoparticles, amorphous solid dispersions, solid dispersions, micronized particles, hydrogels, dendrimers, cyclodextrins, polymer drug conjugates, iron oxide nanoparticle (magnetic carrier), gold nanoparticle

In principle, the composition may be administered orally, e.g. as a pill or liquid, or the composition may be injected into a subject.

A thirteenth aspect relates to a method according to one of aspects 9 to 12, wherein the cancer is selected from the group consisting of solid tumor and non-solid tumors and wherein preferably the cancer is a solid tumor selected from the group consisting of lung cancer, liver cancer, pancreatic cancer, colorectal cancer, breast cancer, prostate cancer, brain cancer, stomach cancer, kidney cancer and cervical cancer. This list, however, is only exemplary and does not serve to limit the present disclosure.

The cancer can also be oral cancer, liver cancer, stomach cancer, colon cancer, breast cancer, lung cancer, bone cancer, pancreatic cancer, skin cancer, head cancer, cervical cancer, skin cancer, cervical cancer, ovarian cancer, colon cancer, small intestine cancer, rectal cancer, fallopian tube carcinoma, anal muscle can-cer, uterus endocrine carcinoma, vaginal carcinoma, vulvar carcinoma, Hodgkin's disease, esophageal cancer, lymph adenocarcinoma, bladder cancer, gallbladder cancer, endocrine cancer, thyroid cancer, parathyroid cancer, adrenal cancer, soft tissue sarcoma, urethral cancer, penile cancer, prostate cancer, chronic Any one or more selected from the group consisting of leukemia, acute leukemia, lympho-cytic lymphoma, kidney cancer, ureteral cancer, renal cell carcinoma, renal pelvic carcinoma, central nervous system tumor, primary central nervous system lym-phoma, spinal cord tumor, brain stem glioma, and pituitary adenoma.

A fourteenth aspect relates to a method according to one of aspects 9 to 13, wherein the saponin agent, i.e. a ginsenoside, is administered at an amount of from 0.001 mg/Kg body weight to 50 mg/Kg body weight. The anthelmin-tic agent, preferably mebendazole, may also be administered at an amount of from 0.001 mg/Kg body weight to 50 mg/Kg body weight. Similarly, the biguanide agent, preferably metformin, may also be administered at an amount of from 0.001 mg/Kg body weight to 50 mg/Kg body weight.

A fifteenth aspect relates to a method according to one of aspects 9 to 14, wherein the effective amount of at least one saponin agent and the effective amount of at least one anthelmintic agent and / or the effective amount of at least one biguanide agent are administered in a single formulation or in at least two separate formulations.

A sixteenth aspect relates to a method according to one of aspects 9 to 15, wherein the effective amount of at least one saponin agent, namely ginsenoside, and the effective amount of at least one anthelmintic agent, namely mebendazole, and / or the effective amount of at least one biguanide agent are administered sequentially or concurrently.

Another, sevententh, aspect of the present disclosure relates to a use of an effective amount of at least one saponin agent, i.e. ginsenoside, and an effective amount of at least one anthelmintic agent, i.e. mebendazole, and / or an effective amount of at least one biguanide agent in the preparation of a composition for the treatment of cancer.

An eighteenth aspect relates to a use according to aspect 17, wherein the biguanide agent is N,N-dimethylbiguanide (metformin).

Another, nineteenth, aspect of the disclosure relates to a kit for treating cancer, preferably a solid tumor, in a human subject, the kit comprising a composition comprising an effective amount of at least one saponin agent, namely ginsenoside, and an effective amount of at least one anthelmintic agent, namely mebendazole, and / or an effective amount of at least one biguanide agent, and instructions for use.

A twentieth aspect of the disclosure relates to a kit according to aspect 19, wherein the effective amount of at least one saponin agent and the effective amount of at least one anthelmintic agent and / or the effective amount of at least one biguanide agent are present in a single formulation or are present in at least two separate formulations.

A twenty-first aspect of the disclosure relates to a method for treating cancer comprising administering to a subject in need thereof (a) an effective amount of at least one saponin agent, namely ginsenoside, and (b) an effective amount of at least one anthelmintic agent, namely mebendazole, to provide a combination therapy having enhanced therapeutic effect and / or reduced side effects compared to the effect of the saponin agent and the an-thelmintic agent each administered alone.

A twenty-second aspect of the disclosure relates to a method for treating cancer comprising administering to a subject in need thereof (a) an effective amount of at least one saponin agent, namely ginsenoside, and (b) an effective amount of at least one biguanide agent, namely mebendazole, to provide a combination therapy having enhanced therapeutic effect and / or reduced side effects compared to the effect of the saponin agent and the biguanide agent each administered alone. The biguanide agent may be administered in combination with the saponin agent and the anthelmintic agent according to the twenty-eighth aspect.

The present disclosure is not limited to the features or aspects of the invention as described above, but also encompasses any such feature or aspect in isolation as well as any combination of features or aspects described above. The present invention is defined in the claims.

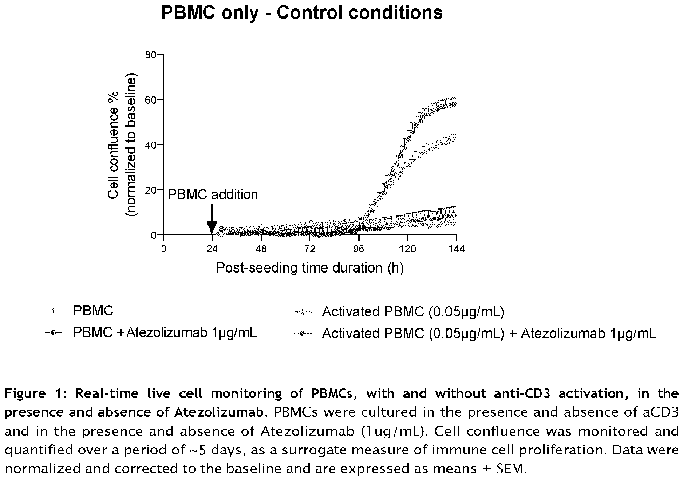

The disclosure is further illustrated by exemplary experimental data that are discussed in the following section. Further features, effects and advantageous become apparent from the following discussion referring to Fig. 1-6.

The following in vitro study was performed in order to evaluate a new array of treatment conditions, including the TLR7/8 agonist (R848), metformin - an antihyperglycemic agent which impairs cellular metabolism (and can thus suppress oncogenic signaling pathways including PI3K/Akt and mTOR signaling pathways), and mebendazole (anti-helminthic agent), for their ability to enhance the killing ability of immune cells towards tumor cells.

The lung A549 tumor cell line was used as target in the immune cell-mediated killing assay described below.

Further experiments are being performed following the same or a highly similar experimental protocol as described in the following section using cell lines from a solid tumor selected from the group consisting of lung cancer, liver cancer, pan-creatic cancer, colorectal cancer, breast cancer, prostate cancer, brain cancer, stomach cancer, kidney cancer and cervical cancer, oral cancer, stomach cancer, colon cancer, bone cancer, pancreatic cancer, skin cancer, head cancer, skin can-cer, ovarian cancer, small intestine cancer, rectal cancer, fallopian tube carcinoma, anal muscle cancer, uterus Endocrine carcinoma, vaginal carcinoma, vulvar carci-noma, Hodgkin's disease, esophageal cancer, lymph adenocarcinoma, bladder cancer, gallbladder cancer, endocrine cancer, thyroid cancer, parathyroid cancer, adrenal cancer, soft tissue sarcoma, urethral cancer, penile cancer, prostate cancer, chronic leukemia, acute leukemia, lymphocytic lymphoma, kidney cancer, ure-teral cancer, renal cell carcinoma, renal pelvic carcinoma, central nervous system tumor, primary central nervous system lymphoma, spinal cord tumor, brain stem glioma, and pituitary adenoma as well as cell lines of non-solid cancers.

For example, the same or similar in vitro studies are being performed on the following non-limiting list of cell lines:
A549 Lung cancer cell line (CDKN2A mutant, K-RAS mutant)
H1299 Lung cancer cell line (TP53-null mutant, NRAS mutant)
MDA-MB-231 Breast cancer cell line (BRAF mutant, CDKN2A mutant, K-RAS mutant, TP53 mutant)
HCT-116 Colon cancer cell line (K-RAS mutant, PIK3CA mutant)
PANC-1 Pancreatic cancer cell line (K-RAS mutant, TP53 mutant, CDKN2A mutant)
SNU-449 Liver cancer cell line (TP53 mutant)

The in vitro study disclosed herein aimed at evaluating the effects of several treatment conditions (with a TLR7/8 agonist (R848), a reference anti-helminthic drug known to display anti-cancer properties (mebendazole), and antihyperglycemic agent (metformin), on the killing capacity of human immune cells - to promote cell death in tumor cell populations.

The experimental conditions that were performed in the study are summarized in the table below:

| **In vitro assessment of the ability to promote the immune cell-mediated tumor killing activity under a number of treatment conditions** | | |
|---|---|---|
| Readouts | On co-culture of activated PBMCs with A549 lung cancer cells: Tumor cell proliferation & apoptosis, & release of 3 cytokines (i.e. IFNg, IL6, and TNFa as agreed with the Sponsor) in cell culture supernatant | |
| | collected 72 hours after co-culture initiation | |
| Method/Platform | Live content imaging (for apoptosis and cell count) & HTRF (for cytokines) | |
| Format | 96 well plates | |
| Tumor cell models | A549 lung tumor cell line | |
| Effector immune cells | 1 human PBMC lot (from a healthy donor) | |
| | | - Untreated |
| | | - Positive control (e.g. Atezolizumab at 1 dose) |
| | | - Ginsenoside Rh2 (50uM) |
| Test conditions | | - R848 (10uM) |
| | | - Mebendazole (1uM) |
| | | - Metformin (1000uM) |
| | | - Ginsenoside Rh2 (50uM)+Mebendazole (1uM) |
| | | - R848 (10uM)+Mebendazole (1uM) |
| | | - Ginsenoside Rh2 (50uM)+Metformin (1000uM) |
| | | - R848 (10uM)+Metformin (1000uM) |
| | | - Mebendazole (1uM)+Metformin (1000uM) |
| | | - Ginsenoside Rh2 (50uM)+Mebendazole (1uM)+Metformin (1000uM) (10uM)+Mebendazole |
| | | - R848 (1uM)+Metformin (1000uM) |

The experimental procedure is described as follows:
1. Test compound treatment: Tested compounds were directly ordered from their Provider, received in powder form and stored at their respective appropriate tem-peratures. Compounds were resuspended according to their respective appropriate conditions. Each of the three compounds were tested at one dose (+ the 0 ; 10µM for R848 ; 50µM for Ginsenoside Rh2, 1000µM for Metformin and 1µM for Meben-dazole), each alone, in combination of two then in combination of three (as de-tailed below). Compound treatments were applied at the time of tumor- immune cell co-culture initiation, in parallel with the addition of the human effector immune cells.
2. Evaluation of test compound effects in an immune cell-mediated A549 lung tumor killing assay: Briefly, A549 human lung carcinoma cell line, modified to ex-press a nuclear fluorescent probe) were seeded at an appropriate cell density and cultured for 24h, before being co-cultured - at the E:T ratio of 10:1 - with human effector immune cells (a healthy donor-originating PBMCs) activated with anti-CD3 antibody (2 doses including the 0: 0 and 0.05µg/mL (the dose was chosen with the Sponsor according to the responsiveness profile of the donor-originating PBMCs), and submitted to the treatment conditions below :
   - Untreated
   - Positive control (e.g. Atezolizumab at 1 dose)
   - Ginsenoside Rh2 (50uM)
   - R848 (10uM)
   - Mebendazole (1uM)
   - Metformin (1000uM)
   - Ginsenoside Rh2 (50uM)+Mebendazole (1uM)
   - R848 (10uM)+Mebendazole (1uM)
   - Ginsenoside Rh2 (50uM)+Metformin (1000uM)
   - R848 (10uM)+Metformin (1000uM)
   - Mebendazole (1uM)+Metformin (1000uM)
   - Ginsenoside Rh2 (50uM)+Mebendazole (1uM)+Metformin (1000uM)
   - R848 (10uM)+Mebendazole (1uM)+Metformin (1000uM)
   Tumor cell proliferation and apoptosis were followed by live cell imaging (based on a nuclear fluorescence and apoptosis specific fluorescence probes). In addition, supernatants from immune / tumor cell co-cultures were retrieved 72h after co-culture initiation & treatment for the quantification of the IFNg, TNFa and IL6 re-leased levels, as surrogates of immune cell activation/activity.
3. Data acquisition and analysis: Image acquisition started 24h after tumor cell seeding, when the treatment with test compounds was applied (at the tumorimmune cell co-culture initiation). Phase contrast, green channel (fluorescent caspase3/7 apoptosis probe) and red channel (fluorescent tumor nuclear probe) images were acquired on an IncuCyte ZOOM^{™} Live cell imager using a 10x objective, with 1 image every 3-4 hours for 5 days monitoring period. Image analysis were performed using IncuCyte ZOOM^{™} software following application of a segmentation mask analysis on phase contrast images to identify cell surface, on red fluorescence images to select tumor cells (expressing the red fluorescent nuclear probe) and on green fluorescence images to identify apoptotic cells (Caspase 3/7 probe ; DEVD-NucView^{™}488). Overlay segmentation analysis was applied to identify apoptotic tumor cells. Data were analyzed and plotted using Graph Pad Prism v6.01 software. In addition, 72h following co-culture initiation and treat-ments, supernatants were collected and effects of test compounds were evaluated on immune cell response activation by mean of the quantification of IFNg, TNFa and IL6 released levels, as key representative surrogates. IFNg, TNFa and IL6 quantification were performed using specific HTRF-based detection kits (TECAN Infinite F500 microplate reader).

This in vitro study particularly aimed at evaluating the effect of four compounds ; a TLR7/8 agonist (R848), metformin - an antihyperglycemic agent, known to impair cellular metabolism (and can thus suppress oncogenic signaling pathways includ-ing PI3K/Akt and mTOR signaling pathways), mebendazole (anti-helminthic agent), and Ginsenoside Rh2 known to induce ER-stress mediated apopto-sis,each alone and in combination of two and three, for their ability to modulate the human immune cell killing activity towards A549 lung cells

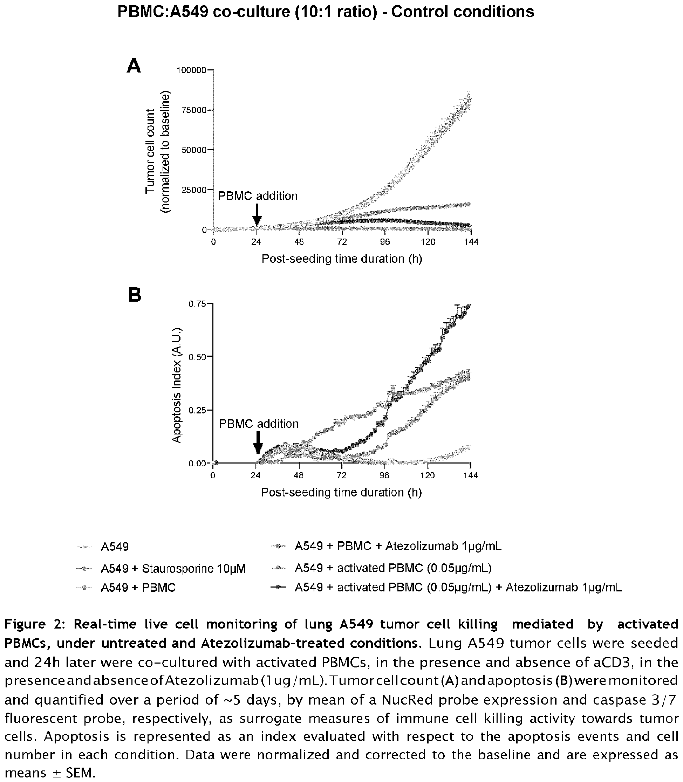

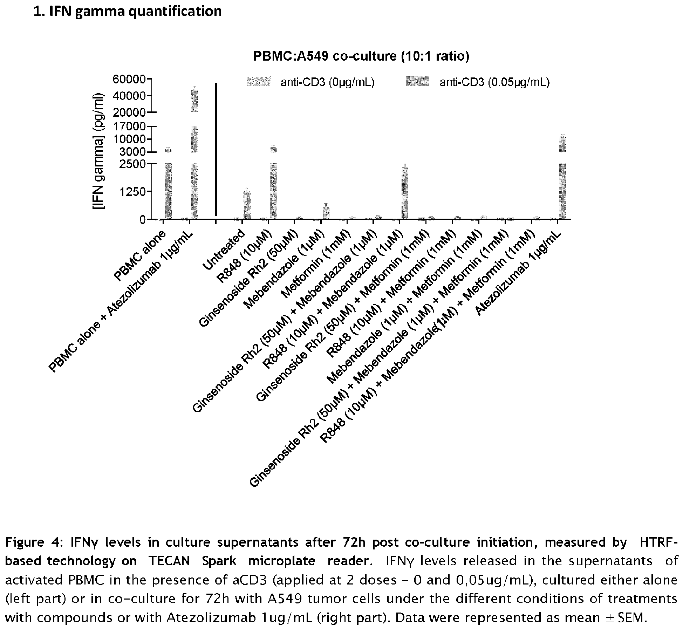

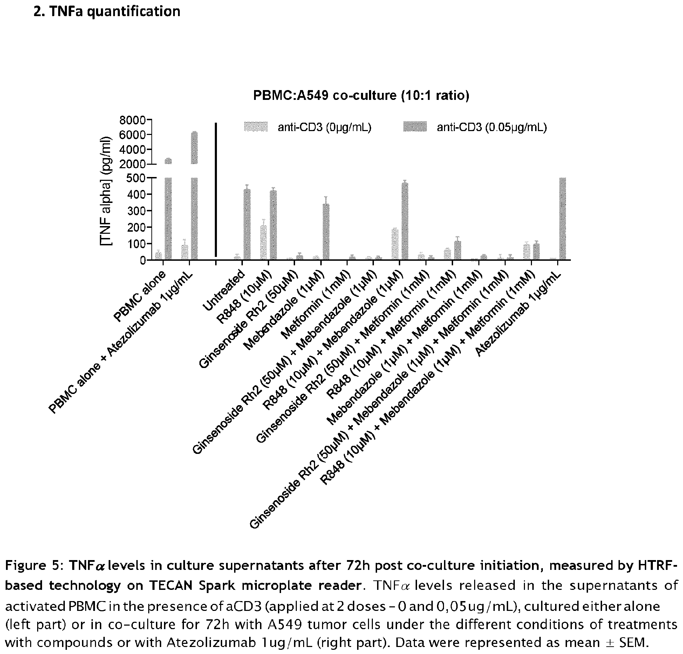

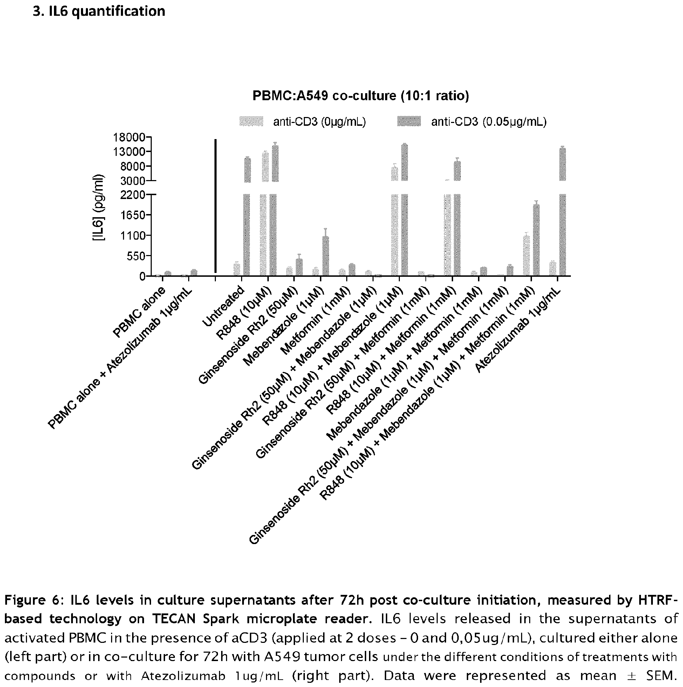

From this study, the following points can be depicted:
- On PBMC cells alone, activated cells were able to proliferate, at the inverse of inactivated cells, and their proliferation was promoted by Atezolizumab (Figure 1).
- In co-cultures for the immune cell mediated tumor killing assay, addition of human PBMCs - without anti-CD3 activation - to A549 tumor cells was shown not to affect the tumor cell proliferation when compared to the A549 tumor cell mono-culture condition. However, and as expected, addition of activated human PBMCs decreased the tumor cell count, an effect visible since 24h-48h after co-culture initiation, along with an appearance of an apoptosis signal (staurosporin treatment showed the apoptosis induction as expected from this positive control). These ef-fects thereby evidencing the killing activity of activated PBMCs were as expected and were shown to tend to be optimized in the presence of Atezolizumab (Figure 2).
- enhanced killing of cancer cells were demonstrated after exposure with ginseno-side and mebendazole and after exposure with ginsenoside and metformin (Fig. 3d). In fact, tumor cell count reached zero or a negligible level between 96-120 af-ter exposure with ginsenoside and mebendazole and after exposure with ginsenoside and metformin (Fig. 3b).
- Furthermore, data obtained through released cytokine quantification showed that PBMC activation/activity and its optimization upon PDL1 blockade were strongly and further confirmed, particularly, by the increased IFNg and TNFa re-leased levels in the supernatants of activated PBMCs either alone and in co-culture with A549 tumor cells (Figures 4 & 5). Indeed, on a hand, activated PBMCs, alone, released higher IFNg levels than non-activated cells, and this release was optimized by atezolizumab (Figure 4). The same was observed with TNFa release, but at a lesser extent modulation (Figure 5).
- both exposure with ginsenoside and mebendazole and exposure with ginseno-side and metformin were shown to lead to very low expressions of TNFa (Fig. 5) and IL6 (Fig. 6) that are usually highly related to side effects of cancer therapies associated with immune checkpoint inhibitors (immuno-oncology), chemothera-py and radiotherapy.

Among the most common inflammatory cytokines as biomarkers of iRAEs is IL-6 which is also known to be a surrogate of immune response, inflammation, tumor progression and pain. Serum IL-6 is associated with worse prognosis and poor survival in cancer patients. Among the first treatments of iRAEs such severe arthri-tis, myocarditis, uveitis, great vasculitis, severe pneumonia, great vasculitis and myasthenia gravis is Tocilizumab (Anti IL-6 Receptor antibody) which inhibits IL-6 signaling. Targeting IL-6 pathway does not activate tumor progression.

Therefore, tumor cell killing without triggering IL-6 and/or activate IL-6 signaling pathways and other inflammatory cytokines pose as great benefit for patients such as inhibiting cancer cachexia, physical and neuropathic pain and fatigue- thus contributing towards improving quality of life. The combination of Ginsenoside and Mebendazole trigger a non-inflammatory, antigen independent immune-mediated tumor cell killing without the secretion of IL-6 and TNF-alpha. Therefore, Ginseno-side and Mebendazole combination reduces cytokine release commonly associated with cancer cachexia, physical and neuropathic pain, fatigue and poor quality of life.
- With respect to treatments with test compounds, cytokine release modulations were observed both in the condition of activated and non-activated PBMCs, but differed, interestingly, depending on the treatment and on the intended cytokine.

∘ Indeed IFNg released levels (Figure 4) were demonstrated to be modulated, more strongly, in the configuration of activated PBMCs. While mebendazole was shown to partially decrease IFNg levels in the supernatants, metformin and Gin-senoside showed a complete inhibition. Conversely, R848 was shown to induce an increase in the IFNg amount when applied alone, an effect which seemed to be (i) partially and (ii) completely "antagonized" by (i) mebendazole and (ii) metfor-min or Ginsenoside Rh2.
∘ As to TNFa levels (Figure 5), also very slight modulations were obtained in the absence of anti-CD3; with R848 showing an increase in the released cytokine, which was inhibited by metformin but not by mebendazole.

In co-cultures with activated PBMCs, there was more TNFa released than with in-activated PBMCs, and these levels were not further increased under R848 or mebendazole treatment. On the other hand, metformin and Ginsenoside Rh2 were shown, each alone or when applied in combination together, to completely abolish this release. While this release was strongly inhibited under metformin in combination with mebendazole, the inhibitory effect of metformin seemed like partially reversed by R848.
o interesting modulations were obtained on IL6 release (Figure 6). While PBMCs alone either inactivated or activated were shown to secrete a very low amount of IL6, this secretion was also shown not to be optimized by PDL1 blockade.

However, IL6 release increased in co-cultures with inactivated PBMCs and even more with activated PBMCs. In both cases, IL6 levels were shown to be slightly optimized by Atezolizumab.

With regards to compounds treatments, in the absence of anti-CD3, R848 interest-ingly displayed a very huge induction of IL6 release, while the three other com-pounds - applied either alone or in combination between them - rather showed an inhibitory action. Also, interestingly, the R848-induced IL6 release was increasing-ly inhibited by mebendazole, metformin, and mebendazole+metformin, respective-ly.

In co-culture with activated PBMCs, IL6 release was very strong in control untreated conditions but remained, slightly, increased by R848 treatment. Also, the inhibitory effects of metformin, Ginsenoside Rh2, and mebendazole were still evidenced, both when they were applied each alone or in combination between them thereby underlining a synergistic inhibition. However, these inhibitory effects were shown, interestingly, to be reversed in the presence of R848, suggesting that TLR7/8 stimulation in the condition of activated immune cells antagonizes the inhibitory modulatory effect of metformin, Ginsenoside Rh2, and mebendazole.

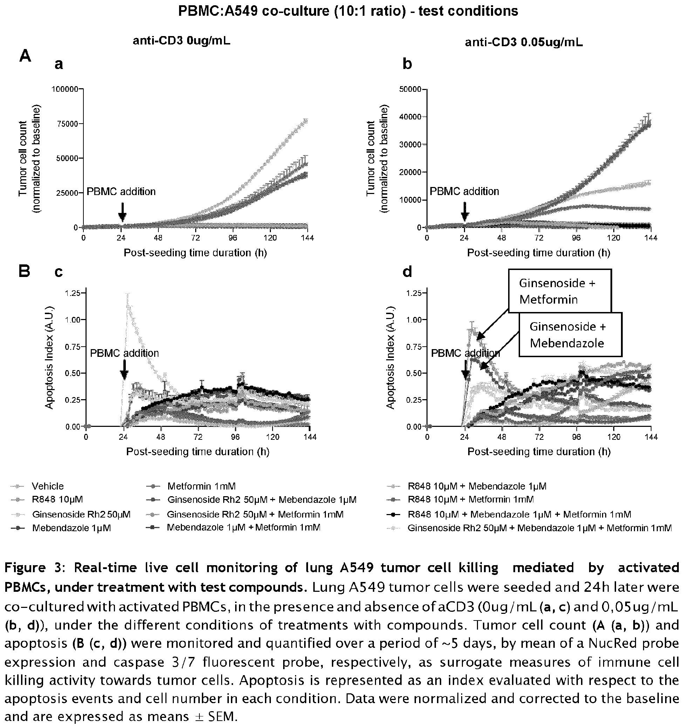

All these effects were supported, and even completed by the kinetic live cell anal-yses of tumor cell count and apoptosis (Figure 3). Indeed, these data showed that:
∘ In the configuration of inactivated PBMCs (Figure 3a&c), all the four com-pounds were shown to promote tumor cell death as indicated by the decrease in tumor cell count and increase in apoptosis signal, with however a stronger effect of mebendazole and Ginsenoside Rh2. The rate of tumor cell death was quite similar under Ginsenoside and metformin combination, and Ginsenoside and mebendazole combination, as with Ginsenoside Rh2 alone. However, a huge increase in tumor cell death was observed under the triple combination thereby suggesting an additive effect between those three compounds.

In addition, while mebendazole and metformin combination, and mebendazole and R848 combination seemed to provide an equivalent tumor cell death rate similar to that induced by mebendazole alone, combination of metformin with R848 showed no particular effect when compared to that of R848 alone. The triple combination showed however an optimized effect trend, particularly likely underlied by mebendazole.
∘ In the configuration of activated PBMCs (Figure 3b&d), R848 was shown to enhance the killing activity of immune cells as stated by the decrease of tumor count and increase in the apoptosis signal. Increase of tumor cell death was also observed under treatment with Ginsenoside Rh2 and mebendazole each applied alone. Also, combinations of mebendazole and Ginsenoside Rh2, and of R848 and mebendazole (slightly) seemed to show synergistic effects translating into an enhanced tumor cell death.

Conversely, metformin application was shown to induce a decrease in tumor cell death. Interestingly, this effect disappeared when metformin was co-applied with mebendazole, and even more, metformin did not seem to impact the effect of mebendazole, while it was able to abolish that of R848. More interestingly, the presence of mebendazole along with R848 was shown to completely reverse the inhibitory metformin effect against R848- enhanced tumor cell death.

Contrary to its effects when combined with R848, metformin was shown to opti-mize the effect of Ginsenoside Rh2, whereas, in the presence of mebendazole, this optimization seemed to be abolished. These last effects with regards to metformin activities also suggest an antagonism between mebendazole and metformin.

As expected, this study thus shows that activated PBMCs promote tumor cell death - an event further optimized upon PDL1 blockade. But more interestingly, it shows that the killing activity of immune cells is likely to be "differently" modulated, and, especially, that regulation of pro/anti-inflammatory TLR7/8 receptor-mediated pathways in myeloid and lymphoid cells is likely to play a primordial role on e.g. antigen presentation and subsequent enhancement of adaptive immune response Results also suggest differential regulations - and synergistic or antagonistic activities - between the tumor and the different immune subpopulations components.

## Claims

1. A composition for use in a method of treating cancer, the composition comprising an effective amount of at least one saponin agent and an effective amount of at least one anthelmintic agent, wherein the saponin agent is a ginsenoside and the anthelmintic agent is a methyl N-(6-benzoyl-1H-benzimidazol-2-yl)carbamate (mebendazole).

2. The composition for use according to one of the preceding claims, further comprising an effective amount of at least one biguanide agent, wherein the biguanide agent preferably is N,N-dimethylbiguanide (metformin).

3. The composition for use according to one of the preceding claims, wherein the composition is a nanocarrier formulation, wherein the nanocarrier preferably is PEGylated or non-PEGylated.

4. The composition for use according to of claim 3, wherein the nanocarrier comprises a liposome, micelles, polymeric nanoparticle, polymeric micelle, dendrimer and / or mesoporous nanoparticles.

5. The composition for use according to one of the preceding claims, wherein the cancer is selected from the group consisting of solid tumor and non-solid tumors and wherein preferably the cancer is a solid tumor selected from the group consisting of lung cancer, liver cancer, pancreatic cancer, colorectal cancer, breast cancer, prostate cancer, brain cancer, stomach cancer, kidney cancer and cervical cancer.

6. The composition for use according to one of the preceding claims, wherein the saponin agent, preferably a ginsenoside, is administered at an amount of from 0.001 mg/Kg body weight to 20 mg/Kg body weight, preferably from 0.01 mg/Kg body weight to 15 mg/kg body weight, in particular from 0.01 mg/Kg body weight to 10 mg/kg body weight.

7. The composition for use according to one of the preceding claims, wherein the effective amount of at least one saponin agent and the effective amount of at least one anthelmintic agent and / or the effective amount of at least one biguanide agent are present in a single formulation or are present in at least two separate formulations.

8. The composition for use according to one of the preceding claims, wherein the effective amount of at least one saponin agent and the effective amount of at least one anthelmintic agent and / or the effective amount of at least one biguanide agent are administered sequentially or concurrently.

9. The composition for use according to one of the preceding claims, wherein the at least one saponin agent and the least one anthelmintic agent and / or the at least one biguanide agent are administered in the same amount.

10. The composition for use according to claim 3, wherein the nanocarrier comprises SMEEDs, SNEDDS, SEDDS, solid lipid nanopartice, nanostructured lipid carrier, microemulsions, liposome, micelles, polymeric nanoparticle, polymeric micelle, dendrimer and / or mesoporous nanoparticles, amorphous solid dispersions, solid dispersions, micronised particles, hydrogels, dendrimers, cy-clodextrins, polymer drug conjugates, iron oxide nanoparticle and / or gold nanoparticles.

## Patentansprüche

1. Eine Zusammensetzung zur Verwendung in einem Verfahren zur Behandlung von Krebs, wobei die Zusammensetzung eine wirksame Menge von mindestens einem Saponin-Wirkstoff und eine wirksame Menge von mindestens einem Anthelminthikum enthält, wobei der Saponin-Wirkstoff ein Ginsenosid ist und das Anthelminthikum Methyl N-(6-Benzoyl-1H-Benzimidazol-2-yl)carbamat (Mebendazol) ist.

2. Die Zusammensetzung zur Verwendung gemäß einem der vorhergehenden Ansprüche, ferner enthaltend eine wirksame Menge von mindestens einem Biguanid-Wirkstoff, wobei der bevorzugte Biguanid-Wirkstoff N,N-Dimethylbiguanid (Metformin) ist.

3. Die Zusammensetzung zur Verwendung gemäß einem der vorhergehenden Ansprüche, wobei die Zusammensetzung eine Nanocarrier-Formulierung ist, wobei der Nanocarrier vorzugsweise PEGyliert oder nicht-PEGyliert ist.

4. Die Zusammensetzung zur Verwendung gemäß Anspruch 3, wobei der Nanocarrier ein Liposom, Mizellen, polymerische Nanopartikel, polymerische Mizellen, Dendrimer und/oder mesoporöse Nanopartikel umfasst.

5. Die Zusammensetzung zur Verwendung gemäß einem der vorhergehenden Ansprüche, wobei der Krebs aus der Gruppe bestehend aus soliden Tumoren und nicht-soliden Tumoren ausgewählt ist und wobei der Krebs vorzugsweise ein solider Tumor ist, der aus der Gruppe bestehend aus Lungenkrebs, Leberkrebs, Bauchspeicheldrüsenkrebs, Darmkrebs, Brustkrebs, Prostatakrebs, Gehirntumor, Magenkrebs, Nierenkrebs und Gebärmutterhalskrebs ausgewählt ist.

6. Die Zusammensetzung zur Verwendung gemäß einem der vorhergehenden Ansprüche, wobei der Saponin-Wirkstoff, vorzugsweise ein Ginsenosid, in einer Menge von 0,001 mg/kg Körpergewicht bis 20 mg/kg Körpergewicht, vorzugsweise von 0,01 mg/kg Körpergewicht bis 15 mg/kg Körpergewicht, insbesondere von 0,01 mg/kg Körpergewicht bis 10 mg/kg Körpergewicht verabreicht wird.

7. Die Zusammensetzung zur Verwendung gemäß einem der vorhergehenden Ansprüche, wobei die wirksame Menge von mindestens einem Saponin-Wirkstoff und die wirksame Menge von mindestens einem Anthelminthikum und/oder die wirksame Menge von mindestens einem Biguanid-Wirkstoff in einer einzelnen Formulierung oder in mindestens zwei separaten Formulierungen vorliegen.

8. Die Zusammensetzung zur Verwendung gemäß einem der vorhergehenden Ansprüche, wobei die wirksame Menge von mindestens einem Saponin-Wirkstoff und die wirksame Menge von mindestens einem Anthelminthikum und/oder die wirksame Menge von mindestens einem Biguanid-Wirkstoff sequenziell oder gleichzeitig verabreicht werden.

9. Die Zusammensetzung zur Verwendung gemäß einem der vorhergehenden Ansprüche, wobei mindestens ein Saponin-Wirkstoff und mindestens ein Anthelminthikum und/oder mindestens ein Biguanid-Wirkstoff in der gleichen Menge verabreicht werden.

10. Die Zusammensetzung zur Verwendung gemäß Anspruch 3, wobei der Nanocarrier SMEEDs, SNEDDS, SEDDS, feste Lipid-Nanopartikel, nanostrukturierte Lipidträger, Mikroemulsionen, Liposomen, Mizellen, polymerische Nanopartikel, polymerische Mizellen, Dendrimer und/oder mesoporöse Nanopartikel, amorphe feste Dispersionen, feste Dispersionen, mikronisierte Partikel, Hydrogels, Dendrimer, Cyclodextrine, Polymer-Wirkstoff-Konjugate, Eisenoxid-Nanopartikel und/oder Gold-Nanopartikel

## Revendications

1. Une composition destinée à être utilisée dans une méthode de traitement du cancer, ladite composition comprenant une quantité efficace d'au moins un agent saponine et une quantité efficace d'au moins un agent anthelminthique, l'agent saponine étant un ginsénoside et l'agent anthelminthique étant du Z 11 méthyl N-(6-benzoyl-1H-benzimidazol-2-yl)carbamate (mébendazole).

2. La composition destinée à être utilisée selon l'une des revendications précédentes, comprenant en outre une quantité efficace d'au moins un agent biguanide, de préférence du N,N-diméthylbiguanide (metformine).

3. La composition destinée à être utilisée selon l'une des revendications précédentes, **caractérisée en ce qu'**elle est une formulation à base de nanotransporteurs, ceux-ci étant de préférence PEGylés ou non PEGylés.

4. La composition destinée à être utilisée selon la revendication 3, **caractérisée en ce que** le nanotransporteur comprend un liposome, des micelles, une nanoparticule polymérique, une micelle polymérique, un dendrimère et/ou des nanoparticules mésoporeuses.

5. La composition destinée à être utilisée selon l'une des revendications précédentes, **caractérisée en ce que** le cancer est choisi dans le groupe comprenant les tumeurs solides et non solides, et de préférence une tumeur solide choisie dans le groupe comprenant le cancer du poumon, du foie, du pancréas, colorectal, du sein, de la prostate, du cerveau, de l'estomac, du rein et du col de l'utérus.

6. La composition destinée à être utilisée selon l'une des revendications précédentes, **caractérisée en ce que** l'agent saponine, de préférence un ginsénoside, est administré à une dose comprise entre 0,001 mg/kg de poids corporel et 20 mg/kg de poids corporel, de préférence entre 0,01 mg/kg de poids corporel et 15 mg/kg de poids corporel, en particulier entre 0,01 mg/kg de poids corporel et 10 mg/kg de poids corporel.

7. La composition destinée à être utilisée selon l'une des revendications précédentes, **caractérisée en ce que** la quantité efficace d'au moins un agent saponine et la quantité efficace d'au moins un agent anthelminthique et/ou la quantité efficace d'au moins un agent biguanide sont présentes dans une seule formulation ou dans au moins deux formulations distinctes.

8. La composition destinée à être utilisée selon l'une des revendications précédentes, **caractérisée en ce que** la quantité efficace d'au moins un agent saponine et la quantité efficace d'au moins un agent anthelminthique et/ou la quantité efficace d'au moins un agent biguanide sont administrées de manière séquentielle ou simultanée.

9. La composition destinée à être utilisée selon l'une des revendications précédentes, **caractérisée en ce que** l'agent saponine, l'agent anthelminthique et/ou l'agent biguanide sont administrés en quantité identique.

10. La composition destinée à être utilisée selon la revendication 3, **caractérisée en ce que** le nanotransporteur comprend des SMEEDs, SNEDDS, SEDDS, des nanoparticules lipidiques solides, un transporteur lipidique nanostructuré, des microémulsions, liposomes, micelles, nanoparticules polymériques, micelles polymériques, dendrimères et/ou nanoparticules mésoporeuses, dispersions solides amorphes, dispersions solides, particules micronisées, hydrogels, dendrimères, cyclodextrines, conjugués polymère-médicament, nanoparticules d'oxyde de fer et/ou nanoparticules d'or.
